# EUROPEAN PATENT APPLICATION

(11) **EP 4 578 445 A1**
(43) Date of publication of application: **02.07.2025**
(21) Application number: 23220376.0
(22) Date of filing: 27.12.2023
(51) Int. Cl.: A61K 31/13, A61K 31/14, A61K 31/19, A61K 31/198, A61K 31/20, A61K 31/21, A61K 31/23, A61K 45/06, A61P 35/00

(54) **COMPOSITION COMPRISING A METHYL GROUP DONOR AND AN ACETYL-COA DONOR FOR USE IN THE PREVENTION OF SKIN CANCER**

(71) Applicant: IUF Leibnitz-Institut für umweltmedizinische Forschung GmbH, 40225 Düsseldorf (DE)
(72) Inventor: Majora, Marc, 40225 Düsseldorf (DE); Krutmann, Jean, 40225 Düsseldorf (DE)
(74) Representative: dompatent von Kreisler Selting Werner - Partnerschaft von Patent- und Rechtsanwälten mbB

(57) **Abstract**

Composition comprising a first compound A, which is a methyl group donor, and a second compound B, which is an acetyl-CoA donor, for use in the prevention of skin cancer.

## Description

The present invention relates to compositions comprising a methyl-group donor compound and an acetyl-CoA donor compound for use in the prevention of skin cancer.

Skin cancer occurs in people of all races and can affect people of any age. Skin cancer is the most commonly diagnosed type of cancer, globally accounting for at least 40% of cancer cases. Ultraviolet (UV) radiation from sun exposure is believed to be the primary cause of skin cancer, however, other factors can play a role including, smoking tobacco, human papillomavirus infections, genetics, chronic non-healing wounds, environmental carcinogens, artificial UV radiation, and light skin color - just to name a few.

While the exact cause of skin cancer is still unknown, UV radiation from sun exposure is believed to be the primary cause. Another cause may be artificial UV radiation, e.g., from sunbeds/tanning beds. While any region of the body may be affected by skin cancer, in children and adults, skin cancer typically occurs on the face, neck, head, and arms.

Skin cancers are often mistaken by patients for non-malignant skin abnormalities, which can result in late detection that leads to difficulties in treating the disease and fatal outcomes. The two most common of skin cancers are basal-cell carcinoma (BCC) and squamous-cell carcinoma (SCC). Together they are referred to as non-melanoma skin cancer (NMSC) and account for 95% of all skin cancers. The precancerous lesion which very frequently develops into SCC is called Actinic Keratosis (AK). In addition, there is malignant melanoma, also called melanoma, which accounts for about 4% of all skin cancers but causes 75% of all skin cancer deaths. Melanoma occurs from melanocytes in the epidermis, many of which are metastatic cancers or carcinomas that lead to death. Another rare form of aggressive skin cancer is Merkel cell carcinoma (MCC).

Basal cell carcinoma is characterized by a raised, smooth, pearly bump on the sun-exposed skin of an individual's head, neck, or shoulders. Often small blood vessels can be seen within the tumor. Crusting of the tumor, as well as bleeding can occur. Individuals sometimes mistake BCC as a sore that will not heal. BCC is the least deadly form of skin cancer and often with proper treatment can be eliminated completely. If untreated, however, it continues to grow and thereby destroys the surrounding tissue.

Squamous-cell carcinoma is characterized by a red, scaling, thickened patch on the sun exposed skin of an individual. Some forms of SCC appear as firm hard nodules and as dome shapes. Breaks and bleeding of the nodules may occur. If left untreated the SCC could develop into a large mass. Also, in some cases SCC causes metastasis.

Actinic keratosis (AK) is the first step in the development of SCC. It is a prominent, rough, scaly skin lesion which results from the proliferation of transformed neoplastic keratinocytes as a consequence of chronic exposure to ultraviolet radiation. It may spontaneously regress but very frequently it remains stable and/or transforms into invasive SCC which may even metastasize. Importantly, skin with AK, even when clinically looking normal, shows an accumulation of oncogenic mutations leading to a proliferation of atypical keratinocytes and thereby the development of more AK and, in particular, SCC. This is called field cancerization.

Malignant melanoma is characterized by shades or brown to black lesions. There are also some malignant melanomas which appear pink, red or flesh color, these are called amelanotic melanomas. The amelanotic melanomas are a more aggressive form of melanoma. Some of the warning signs of malignant melanoma could include changes in size, shape, color, elevation of a mole, the development of a new mole in the transitional period from puberty to adulthood, itching, ulceration or bleeding. Melanoma is the deadliest form of skin cancer.

Merkel cell carcinoma is characterized by rapid growing, non-tender flesh colored to red/violet bumps that are usually not painful or itchy. These bumps appear on the highly sun exposed skin of the head, neck and arms. Individuals often mistake MCC for a cyst or other type of cancer.

The current treatment of skin cancer can be invasive and arduous. Treatment for skin cancer typically involves both lifestyle changes and the use of undesirable medications. In some cases of basal-cell carcinoma, squamous-cell carcinoma or actinic keratosis, several types of skin cancer treatment options may be given, including surgery, topical chemotherapy, photodynamic therapy, or radiation therapy. All of these have side effects and cause different degrees of skin irritations and pain sensations. In cases of melanoma, treatment may include surgery, chemotherapy, isolated limb perfusion, immunotherapy, and radiation therapy. However, some of these treatments are replete with drawbacks such as flu-like symptoms, extreme fatigue, hair-loss, DNA damage, development of secondary cancer, radiation burns in the skin, and cell migration into the bloodstream.

While the above-identified medical treatments do appear to provide at least treatment to those with skin cancer, such treatment remains non-desirous and/or problematic because, among other things, none of the above-identified treatments provide sufficient results from the debilitating effects of skin cancer without material drawbacks.

Reducing the incidence of potentially dangerous skin cancers would not only reduce their potentially severe morbidity and mortality, but also dramatically reduce the high costs typically associated with surgical and medical treatments. It is therefore highly desirable to prevent the development of skin cancer, including its precancerous stages.

One possibility for trying to prevent the development of skin cancer is the use of sunscreen, also known as sunblock or sun cream. Sunscreen is a photoprotective topical product which comes as lotions, sprays, gels, foams (such as an expanded foam lotion or whipped lotion), sticks, powders, and other topical products. Medical organizations such as the American Cancer Society recommend the use of sunscreen because it aids in the prevention of squamous-cell carcinomas. The routine use of sunscreens may also reduce the risk of melanoma. To effectively protect against all the potential damages of UV light, the use of broad-spectrum sunscreens (covering both UVA and UVB radiation) has been recommended.

Problems associated with the application of sunscreen products are that some products have a low acceptance with the consumers because of their consistency and appearance after application to the skin (such as white residues or oily skin). Moreover, consumers often use lower amounts than recommended.

Therefore, there is the need to provide further products which can be used in the prevention of skin cancer. The object of the present invention is thus to provide compositions for use in the prevention of skin cancer.

The object of the invention is surprisingly solved by a composition comprising a first compound A, which is a methyl group donor, and a second compound B, which is an acetyl-CoA donor, for use in the prevention of skin cancer.

Surprisingly, it has been found that a combination of a methyl group donor and an acetyl-CoA donor can induce the skin's natural protection mechanism against UV radiation. To protect itself against UV radiation, human skin has developed the ability to tan. Tanning results from the production of melanin within the epidermal compartment of skin. This mechanism is called melanogenesis. Specifically, two main types of melanin, eumelanin and pheomelanin, are produced by human melanocytes residing in the epidermis. Melanogenesis is triggered primarily by UVB radiation. The UVB radiation triggers an increase in production of melanin, which is the body's reaction to direct nuclear DNA damage (formation of pyrimidine dimers) from UV irradiation. Both types of melanin, by virtue of their capacity to absorb UV radiation, serve to protect surrounding skin cells, such as keratinocytes, against UV radiation-induced damage of nuclear DNA, which, if unrepaired, might cause mutations in genes which regulate the growth of such cells such as oncogenes and tumor suppressor genes and thereby lead to the development of skin cancer. In addition, both types of melanin have antioxidative properties and thereby additionally protect human skin from oxidative damage, which might be caused by exposure to UV radiation. The potency of melanin synthesis to prevent skin cancer is being proven by the well-established fact that individuals with fair skin, which easily develop a sunburn and never or only poorly develop a tan, are significantly more prone to develop skin cancer as compared to darker skin individuals who rarely develop sunburns, but instead tan very well.

Surprisingly, it has been found that topical administration of a methyl group donor in combination with an acetyl-CoA donor can induce melanogenesis in human skin without the need of additional UV irradiation. This causes a tanning response, which results from an increase in epidermal melanin content and thus mimics the natural photoprotection strategy of human skin and provides a tan which is indistinguishable from a natural tan induced by UV radiation, but which does not require exposure of human skin to UV radiation and thus avoids UV radiation-induced health damage. By mimicking the natural photoprotection strategy of the human skin by administering a methyl group donor in combination with an acetyl-CoA donor, the development of skin cancer can advantageously be prevented. Advantageously, the prevention of the development of skin cancer includes the prevention of the development of precancerous stages such as actinic keratosis.

The composition for use in the prevention of skin cancer of the present invention comprises a first compound A, which is a methyl group donor, and a second compound B, which is an acetyl-CoA donor.

Within the meaning of the present invention, "prevention of skin cancer" means that the development of skin cancer can be prevented. Individuals treated with the composition for use of the invention consequently have a significantly reduced risk of developing skin cancer. "Prevention of skin cancer" within the meaning of the present invention also includes the prevention of the development of precancerous lesions. The composition for use of the present invention is thus also suitable to prevent the development of precancerous stages, especially actinic keratosis (AK), a pre-stage of SCC. Therefore, "prevention of skin cancer" within the meaning of the present invention may also be referred to as the "prevention of skin cancer and its pre-stages".

As compound A, any tolerable methyl group donor known from the prior art may be employed. The term "methyl group donor" or "methyl donor" is well known to the person skilled in the art. The term is used in the art to describe compounds which can provide methyl groups in the human body, e.g., in metabolic pathways or DNA synthesis. By way of example, it is pointed to F. Depeint et al. (2006), "Mitochondrial function and toxicity: Role of B vitamins on the one-carbon transfer pathways", Chemico-Biological Interactions, 163, 113-132, and to E.N. Proshkina (2020), "Key Molecular Mechanisms of Aging, Biomarkers, and Potential Interventions", Molecular Biology, 54, 6, 777-811. Exemplary methyl group donors are trimethyl ammonium compounds such as choline and betaine, methionine, folic acid, folate, 5-methyltetrahydrofolate, vitamins B2, B6 and B12, sarcosine, serine, trimethylamine-N-oxide, and S-adenosylmethionine (SAM).

In a preferred embodiment, the first compound A is selected from the group consisting of compounds comprising trimethylamine-groups, sarcosine, trimethylamine-N-oxide, serine, and mixtures of two or more of them. Preferably, the first compound A may be selected from trimethyl ammonium compounds and mixtures of two or more of them. Preferred compounds comprising trimethylamine-groups are choline and betaine. Particularly preferred, the first compound A is selected from choline and betaine. Most preferred is choline as first compound A.

In another preferred embodiment, the first compound A is selected from the group consisting of choline, betaine, sarcosine, trimethylamine-N-oxide, serine, and mixtures of two or more of them, more preferably selected from choline and betaine.

In another preferred embodiment, the first compound A is choline.

As compound B, any tolerable acetyl-CoA donor known from the prior art may be employed. The term "acetyl-CoA donor" is well known to the person skilled in the art. It is used in the art to describe substances or compounds from which acetyl-CoA can be generated in the human body, or substances suppressing compounds which hinder the generation of acetyl-CoA. By way of example, it is pointed to J.M. Frans Trijbels et al. (2004), "Chapter 5 - Biochemical Diagnosis of OXPHOS Disorders" in "Oxidative Phosphorylation in Health and Disease", Eurekah.com and Kluwer Academic / Plenum Publishers. Mixtures of two or more different acetyl-CoA donors can be employed as well. Within the meaning of the present invention, acetyl-CoA donors are substances and compounds from which acetyl-CoA can be generated in the human body, or substances suppressing compounds, such as enzymes, which hinder the generation of acetyl-CoA. Exemplary acetyl-CoA donors are fatty acids, fatty acid derivatives, salts of fatty acids, salts of fatty acid derivatives, triglycerides, triglyceride derivatives, dicarboxylic acid derivatives, and pyruvate dehydrogenase kinase inhibitors.

A compound hindering the generation of acetyl-CoA is for example pyruvate dehydrogenase kinase (PDK). Pyruvate dehydrogenase kinase is a kinase enzyme which acts to inactivate the enzyme pyruvate dehydrogenase by phosphorylating it using ATP. There are four known isozymes of PDK in humans which all catalyze the same reaction. Accordingly, suitable compounds B are also inhibitors of pyruvate dehydrogenase kinases, for example dichloroacetate, AZD7545 ((*R*)-4-(3-Chloro-4-(3,3,3-trifluoro-2-hydroxy-2-methylpropan-amido)phenylsulfonyl)-N,N-dimethylbenzamide), PS10 (2-[(2,4-Dihydroxyphenyl)sulfonyl]isoindoline-4,6-diol), dicoumarol, JX06 (Bis(morpholinothio-carbonyl)disulfide), leelamine or VER-246608 (N-[4-(2-chloro-5-methyl-4-pyrimidinyl)phenyl]-N-[[4-[[(2,2-difluoroacetyl)amino]methyl]phenyl]methyl]-2,4-dihydroxy-benzamide).

Fatty acids within the meaning of the present invention are carboxylic acids with 2 to 28 carbon atoms, especially 10 to 25 carbon atoms. Their chain is aliphatic and can be saturated or unsaturated, linear, or branched. Within the meaning of the present invention, both acetic acid and butyric acid are fatty acids. Salts of fatty acids within the meaning of the present invention are pharmaceutically acceptable salts of fatty acids. Any pharmaceutically acceptable salt of the above-mentioned fatty acids known from the prior art may be employed. Suitable salts are e.g., sodium salts of fatty acids, potassium salts of fatty acids, ammonium salts of fatty acids, calcium salts of fatty acids or magnesium salts of fatty acids.

Fatty acid derivatives within the meaning of the present invention are modified fatty acids, such as oxylipins, hydroxy fatty acids, diols, alkenones, and wax esters. β-hydroxybutyrate is an example for a fatty acid derivative which may be used in the present invention. Salts of fatty acid derivatives within the meaning of the present invention are pharmaceutically acceptable salts of fatty acid derivatives. Any pharmaceutically acceptable salt of the above-mentioned fatty acid derivatives known from the prior art may be employed. Suitable salts are e.g., sodium salts of fatty acid derivatives, potassium salts of fatty acid derivatives, ammonium salts of fatty acid derivatives, calcium salts of fatty acid derivatives or magnesium salts of fatty acid derivatives.

In a preferred embodiment of the invention, the second compound B is selected from fatty acids, fatty acid derivatives, salts of fatty acids, salts of fatty acid derivatives, triglycerides, triglyceride derivatives, dicarboxylic acid derivatives, and pyruvate dehydrogenase kinase inhibitors, particularly from acetate, butyrate, triheptanoin, dimethyl-α-ketoglutarate, and pyruvate dehydrogenase kinase inhibitors. Acetate and butyrate are the salts of acetic acid and butyric acid, respectively. Triheptanoin is an example for a triglyceride which may be used in the present invention. Dimethyl-α-ketoglutarate is an example for a dicarboxylic acid derivative which may be used in the present invention.

In another preferred embodiment, the second compound B is selected from acetate, butyrate, triheptanoin, and dimethyl-α-ketoglutarate, preferably selected from acetate and butyrate.

In another preferred embodiment, the second compound B is acetate.

In a preferred embodiment, the inventive composition for use consists of the first compound A and the second compound B.

A particularly preferred inventive composition for use comprises choline as compound A and an acetate as compound B, or consists thereof.

Another particularly preferred inventive composition for use comprises choline as compound A and a butyrate as compound B, or consists thereof.

Still another particularly preferred inventive composition for use comprises choline as compound A and triheptanoin as compound B, or consists thereof.

Still another particularly preferred inventive composition for use comprises choline as compound A and dimethyl-α-ketoglutarate as compound B, or consists thereof.

Preferably, the molar ratio of the compound A to compound B is in the range of from 10:1 to 1:10, preferably from 8:1 to 1:8, more preferably from 5:1 to 1:5, even more preferably from 2.5:1 to 1:2.5.

In another preferred embodiment, the molar ratio of the compound A to compound B is in the range of from 10:1 to 1:1, preferably from 8:1 to 2:1, more preferably from 5:1 to 2.5:1.

In a preferred embodiment, the concentration of compound A is in the range of from 1 to 250 mM, preferably in the range of from 5 to 200 mM, more preferably in the range of from 10 to 100 mM, even more preferably in the range of from 25 to 50 mM.

In a preferred embodiment, the concentration of compound B is in the range of from 1 to 100 mM, preferably in the range of from 5 to 50 mM.

In another preferred embodiment, the concentration of compound A is in the range of from 1 to 250 mM, preferably in the range of from 5 to 200 mM, more preferably in the range of from 10 to 100 mM, even more preferably in the range of from 25 to 50 mM, and the concentration of compound B is in the range of from 1 to 100 mM, preferably in the range of from 5 to 50 mM.

In another preferred embodiment, the concentration of compound A is in the range of from 10 to 100 mM and the concentration of compound B is 10 mM.

The dosage of the inventive composition for use and the components therein varies depending on the type of effect desired, on the weight, age, sex of the subject, and the method of administration. Generally, compositions for use can be administered in an amount based on the average weight of a subject.

In a preferred embodiment, the inventive composition for use is administered in an amount of 1 to 250 mg/kg/d, preferably 2 to 240 mg/kg/d, more preferably 5 to 220 mg/kg/d, of compound A and 1 to 250 mg/kg/d, preferably 10 to 220 mg/kg/d, more preferably 50 to 200 mg/kg/d, of compound B.

In a further preferred embodiment, the inventive composition for use is administered in an amount of 1 to 25 mg/kg/d, preferably 2 to 20 mg/kg/d, more preferably 5 to 10 mg/kg/d, even more preferably 7 to 8 mg/kg/d, of choline as compound A and 1 to 250 mg/kg/d, preferably 10 to 220 mg/kg/d, more preferably 50 to 200 mg/kg/d, of compound B. Preferably, compound B is acetate.

In a further preferred embodiment, the inventive composition for use is administered in an amount of 100 to 250 mg/kg/d, preferably 120 to 240 mg/kg/d, more preferably 130 to 220 mg/kg/d, of betaine as compound A and 1 to 250 mg/kg/d, preferably 10 to 220 mg/kg/d, more preferably 50 to 200 mg/kg/d, of compound B. Preferably, compound B is acetate.

In a preferred embodiment, the inventive composition for use is administered every day.

The inventive composition for use may be administered by any suitable method known from the prior art. In a preferred embodiment, the inventive composition for use is administered orally, intravenously, or topically, preferably topically. The inventive composition for use may be administered topically as compound in skincare products, preferably in sunscreen products. Depending on the administration, the composition for use according to the present invention may comprise any suitable additive known by the person skilled in the art, such as solvents, etc. For topical applications, antioxidants, DNA-repair-enzymes, vitamins, UV-filters, pre- or probiotic substances, etc. may be included.

The skin cancer may be skin cancer caused by ultraviolet (UV)-light irradiation. Preferably, the skin cancer is selected from the group consisting of basal-cell carcinoma (SCC), squamous-cell carcinoma (SCC), malignant melanoma, Merkel cell carcinoma (MCC), Kaposi's sarcoma, keratoacanthoma, sebaceous carcinomas, atypical fibroxanthoma, leiomyosarcoma, angiosarcoma, and porocarcinoma, more preferably selected from the group consisting of basal-cell carcinoma (SCC), squamous-cell carcinoma (SCC), and malignant melanoma.

In a preferred embodiment, the first compound A in combination with the second compound B induces melanogenesis in skin cells, especially in melanocytes, without the need of additional UV irradiation. This enables the prevention of skin cancer. Although an additional UV irradiation is not needed, it is still possible in order to increase the beneficial effects of the treatment with the first compound A in combination with the second compound B.

In the following the rationale of the invention is further explained and it is elaborated, how the inventive composition for use is linked to the prevention of skin cancer. These theoretical findings are to be understood as illustrative only and the present invention is not intended to be bound by the following theory.

### Brief description of the Figures

Fig. 1 shows microscope images of skin sections of skin samples obtained from an aged donor, which were cultivated *ex vivo* and treated with various concentrations of choline (10 - 100 mM) and a fixed concentration of acetate (10 mM) or received control medium according to Example 1.
Fig. 2 shows a Western Blot analysis of proteins isolated from *ex vivo* cultivated skin samples obtained from an aged donor, which were treated with various concentrations of choline (10 - 100 mM) and a fixed concentration of acetate (10 mM) or received control medium according to Example 2.
Fig. 3 shows the relative gene expression level of Tyrp1 in human PIG1 melanocytes, which were treated with various concentrations of choline (10 - 100 mM) and a fixed concentration of acetate (10 mM), or which received control medium, and were either irradiated with UVB or left unirradiated according to Example 3.

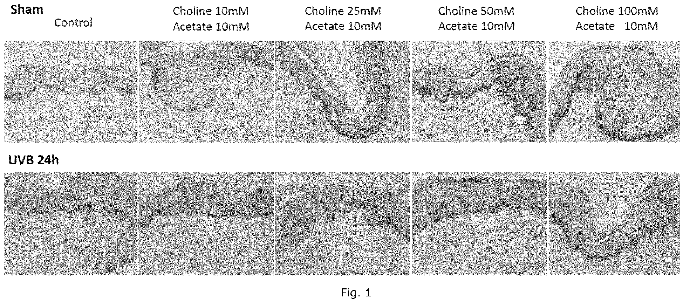

Surprisingly, it has been found that topical administration of a methyl group donor in combination with an acetyl-CoA donor can induce melanogenesis in human skin without the need of additional UV irradiation. Topical application of the inventive composition for use to human skin from healthy, adult donors caused a significant increase in epidermal melanin content. Accordingly, staining of histological skin sections using a Fontana Masson protocol revealed that treatment with the inventive composition for use increased melanin levels in a dose-dependent manner (Fig. 1). Of note, this tanning response could be elicited without any additional UV irradiation, or, in other words, in unirradiated human skin. A tanning response can also be achieved in UV-preirradiated skin, i.e., application of the inventive composition for use is capable to further enhance UV-radiation-induced melanin content within the epidermis.

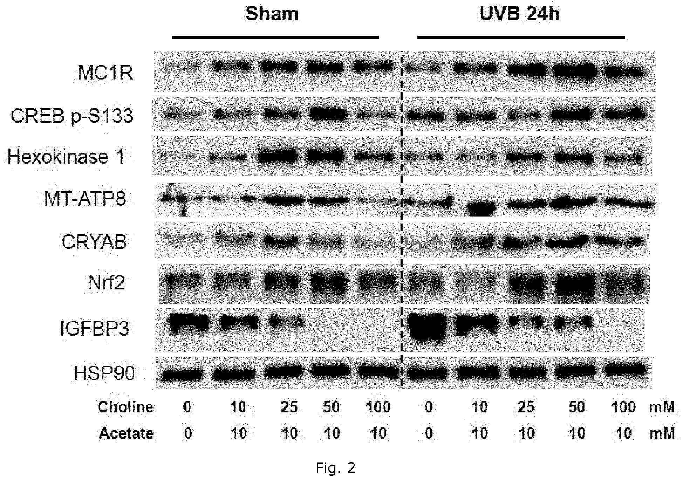

The increase in melanin production was associated with an increased expression of proteins which are known to be critically involved in *de novo* synthesis of melanin in human skin. Accordingly, increased protein levels of the melanocortin 1 receptor (MC1R) and the phosphorylated form of CREB (p-S133) which are both involved in melanin synthesis were found (Fig. 2). Taken together these results show that topical application of the inventive composition for use can induce a tanning response in human skin. This tanning response is due to an increase in epidermal melanin content, indicating that human skin treated by the composition for use of the invention is better protected against UV radiation-induced DNA damage, and thus better protected against the development of skin cancer.

Melanin additionally protects human skin against UV radiation and other environmental threats by acting as an antioxidant. Surprisingly, it was additionally observed that topical application of the composition for use of the invention causes the increased expression of another antioxidative system in human skin, i.e., the transcription factor Nrf2 which acts as a cytoprotective master regulator of cellular redox homeostasis and stress responses. Nrf2 was upregulated by the composition for use of the invention in unirradiated as well as UV-irradiated human skin at the protein level (Fig. 2).

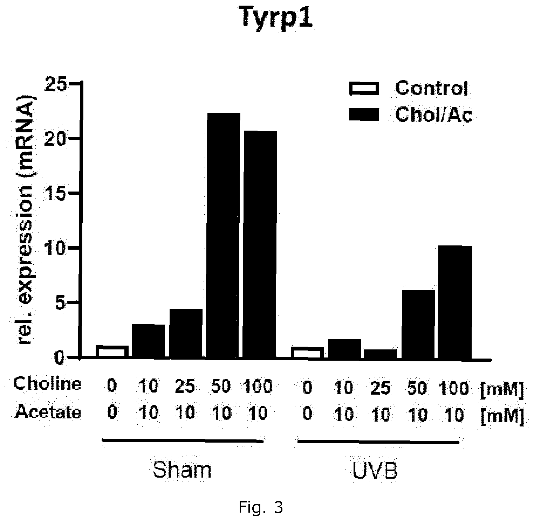

Surprisingly, the combined application of the composition for use of the invention may also cause melanin synthesis in human melanocytes by modulating the expression of Tyrp1, a gene responsible for the synthesis of the UV-protective pigment eumelanin in melanocytes (Fig. 3).

It is to be understood that each preferred embodiment and each preferred feature can be combined with each other without further limitation.

In the following examples, the present invention is disclosed but without limiting the scope of protection to these specific examples.

### EXAMPLES

### Description of the materials and methods used in Examples 1 to 3

### Ex vivo skin culture and treatment

A human skin sample derived from a female 57-year-old donor undergoing skin surgery was received from a local hospital. After being placed under a laminar flow hood, the skin sample was shortly disinfected in 70% EtOH, washed three times and kept in phosphate buffered saline (PBS) containing 100 U/ml penicillin, 100 µg/ml streptomycin and 0.25 µg/ml amphotericin B. The skin sample was cut into smaller pieces of equal size and the subcutaneous fat tissue was removed using scalpels and scissors.

For skin culture, a sterile surgical SMI Spon gelatin sponge (SMI AG, Belgium) was cut into small equal pieces using a scalpel. The sponge pieces were put into six-well-plates and allowed to soak with culture medium [DMEM / Ham's F12-Medium (1:1), 2% FCS, 1x Glutamax, 1x antibiotics/antimycotics] containing varying concentrations of choline chloride (10, 25, 50 or 100 mM) and a constant concentration of sodium acetate (10 mM) (both chemicals were purchased from Sigma-Aldrich). Control medium used here did not contain any added choline chloride or sodium acetate. Skin pieces were placed on the surface of the soaked sponges and cultivated for 24 h.

For UVB exposure, culture medium was removed and skin pieces were irradiated with UVB (dose 50 mJ/cm²). After irradiation, fresh corresponding medium was added and the skin pieces were cultivated for additional 24 h.

### Histological processing and Fontana Masson staining

Following the treatment, skin pieces were fixed in 4% paraformaldehyde at 4 °C overnight. After being dehydrated, skin samples were embedded in paraffin and cut into thin sections using a microtome. Fontana Masson staining was done using a commercially available kit (ScyTek Laboratories, USA) according to the protocol of the manufacturer. Briefly, sections were deparaffinized in citrate buffer and rehydrated before being incubated in ammoniacal silver solution at 60 °C for 45 min. After three times of washing in distilled H₂O, slides were incubated in gold chloride solution (0.2%) for 30 sec. Slides were again washed three times with distilled water before being incubated in sodium thiosulfate solution (5%) for 2 min. After being rinsed under running tap water for 2 min, slides were washed twice with distilled water. Sections were counterstained using Nuclear Fast Red solution for 5 min, rinsed for 2 min under running tap water and washed twice with distilled water. Using three changes of absolute ethanol, sections were dehydrated before being mounted in synthetic resin and being analyzed with a light microscope.

### Protein extraction and Western blot analysis

After treatment, skin pieces were transferred into 2 ml Eppendorf tubes filled with ice cold RIPA lysis and protein extraction buffer supplied with proteinase and phosphatase inhibitors. A small stainless-steel bead (diameter: 3 mm) was added to each tube and the samples were shredded using a TissueLyser II (Qiagen). Samples were kept on ice for 30 minutes before being spun down at 14000 rpm for 15 minutes at 4 °C. Supernatants with solubilized proteins were collected, snap frozen in liquid nitrogen and stored at -80 °C. For Western Blot detection, protein aliquots were separated by SDS-polyacrylamide gel electrophoresis before being transferred on PVDF membranes by using the Trans-Blot Turbo Transfer system (Bio-Rad). Membrane slices were incubated with the indicated antibodies overnight on a shaker at 4 °C. The following antibodies were used: CREB p-S133 (Cell Signaling, #9198), CRYAB (Novus, #NBP1-22410), Hexokinase 1 (Cell Signaling, #2024), HSP90 (Cell Signaling, #4877), IGFBP3 (Cell Signaling, #64143), MC1R (Invitrogen, #PA5-21911), MT-ATP8 (Genetex, #GTX55993), Nrf2 (Novus, #NBP1-32822). After washing, membrane slices were incubated with appropriate secondary antibodies coupled to horseradish peroxidase for 2 hours at room temperature. After washing, membrane slices were incubated with ECL western blotting substrate and protein bands were detected with an Odyssey XF Imaging system (LI-COR).

### Cell Culture and treatment

Human PIG1 melanocytes were grown on culture dishes in melanocyte growth medium containing 5% FCS. 48 hours before UVB irradiation, medium was removed, cells were washed with phosphate buffered saline (PBS) and received growth medium (Ham's F-10 containing 10 ng/ml Tetradecanoyl-Phorbol 13-Acetat (TPA), 100 µM IBMX, 1% Ultroser G and 1x antibiotics/antimycotics). 24 hours later, medium was removed and cells received medium containing varying concentrations of choline chloride (10, 25, 50 or 100 mM) and a constant concentration of sodium acetate (10 mM). Control medium used here did not contain any extra choline chloride or sodium acetate. 24 hours later, medium was removed, cells were washed with PBS and were irradiated with UVB (dose: 60 mJ/cm²) in fresh PBS. Unirradiated control (sham) cells were treated essentially equal but were not exposed to UVB. After irradiation, PBS was removed, cells were treated with the corresponding medium and were cultivated for additional 24 hours.

### RNA isolation & Real-Time PCR

24 hours after irradiation, medium of PIG1 melanocytes was removed, cells were washed with PBS and RNA was extracted using a Direct-zol RNA kit (Zymo Research) according to the protocol of the manufacturer and stored at -80 °C. 200 ng of purified RNA were used for cDNA synthesis using SuperScript reverse transcriptase (Thermo Fisher). Expression level of the gene Tyrp1 was analyzed using a CFX Connect Real-Time PCR detection system and SYBR Green Supermix (both from Bio-Rad). Expression level of Tyrp1 was normalized in comparison to the housekeeper gene TFPI2.

### Example 1

Skin samples obtained from an aged donor were cultivated *ex vivo* and treated with various concentrations of choline (10 - 100 mM) and a fixed concentration of acetate (10 mM) or received control medium. After 48 h of cultivation, skin samples were irradiated with UVB or left unirradiated. 24 h later skin samples were fixed in paraformaldehyde and embedded in paraffin before being cut into sections. The skin sections were subjected to a Fontana Masson staining protocol using a commercially available kit (detailed description of the experimental procedure: see above).

The images shown in Fig. 1 indicate a dose-dependent increase of melanin content by treatment with choline/acetate with and without UVB irradiation. The higher the dose of choline/acetate, the more cells within the respective skin sections show dark brown or black color. The cells with the dark brown or black color located in the bottom layer (the stratum basale) of the epidermis are the melanocytes, and the more intense their color, the more melanin was produced by melanogenesis. Surprisingly, the melanocytes of the skin sample treated with 10 mM choline and 10 mM acetate, but not irradiated with UVB, have a similar amount of melanin like the melanocytes of the skin sample not treated with choline and acetate, but irradiated with UVB.

### Example 2

Skin samples obtained from an aged donor were cultivated *ex vivo* and treated with various concentrations of choline (10 - 100 mM) and a fixed concentration of acetate (10 mM) or received control medium. After 48 h of cultivation, skin samples were irradiated with UVB or left unirradiated and proteins were extracted 24 h after irradiation. Proteins were separated by SDS-polyacrylamide gel electrophoresis and transferred to a PVDF membrane which then was probed with antibodies raised against the indicated markers (detailed description of the experimental procedure: see above).

As shown in Fig. 2, MC1R and CREB p-S133, which are involved in the synthesis of melanin, are increased by choline/acetate, in particular by a choline concentration of 50 mM (in combination with 10 mM acetate) and without UVB irradiation. Hexokinase 1, MT-ATP8, CRYAB and Nrf2 are in particular upregulated at choline concentrations of 25 and 50 mM (in combination with 10 mM acetate, respectively) in both UVB-irradiated and non-irradiated skin samples. The senescence-associated marker IGFBP3 is increased after UVB irradiation and shows a dose-dependent downregulation with and without UVB. HSP90 was used as reference.

### Example 3

Human PIG1 melanocytes were treated with varying concentrations of choline (10 - 100 mM) and a constant concentration of acetate (10 mM). Control cells just received medium without any added choline and acetate. 24 h later, cells were either irradiated with UVB or left unirradiated (Sham). Expression of the melanocyte-associated gene Tyrp1 was analyzed 24 h after UVB irradiation in relation to the housekeeping gene TFPI2 (detailed description of the experimental procedure: see above).

As shown in Fig. 3, choline/acetate leads to a dose-dependent increase of Tyrp1 RNA levels in human PIG1 melanocytes with and without UVB irradiation. This effect is especially pronounced in non-irradiated cells. Tyrp1 is responsible for the synthesis of the UV-protective pigment eumelanin in melanocytes. Thus, these data indicate that choline/acetate may directly affect melanin synthesis in human melanocytes by modulating the expression of Tyrp1.

## Claims

1. Composition comprising a first compound A, which is a methyl group donor, and a second compound B, which is an acetyl-CoA donor, for use in the prevention of skin cancer.

2. Composition for use according to claim 1, wherein the first compound A is selected from the group consisting of compounds comprising trimethylamine-groups, sarcosine, trimethylamine-N-oxide, serine, and mixtures of two or more of them, preferably selected from trimethyl ammonium compounds and mixtures of two or more of them, especially selected from choline and betaine.

3. Composition for use according to claim 1 or 2, wherein the second compound B is selected from fatty acids, fatty acid derivatives, salts of fatty acids, salts of fatty acid derivatives, triglycerides, triglyceride derivatives, dicarboxylic acid derivatives, and pyruvate dehydrogenase kinase inhibitors, particularly from acetate, butyrate, triheptanoin, dimethyl-α-ketoglutarate, and pyruvate dehydrogenase kinase inhibitors.

4. Composition for use according to any one of claims 1 to 3, wherein the second compound B is acetate or butyrate.

5. Composition for use according to any one of claims 1 to 4, wherein the molar ratio of compound A to compound B is from 10:1 to 1:10, preferably from 8:1 to 1:8, more preferably from 5:1 to 1:5, even more preferably from 2.5:1 to 1:2.5.

6. Composition for use according to any one of claims 1 to 5, wherein the composition is administered orally, intravenously, or topically.

7. Composition for use according to any one of claims 1 to 6, wherein the composition is administered topically.

8. Composition for use according to claim 6 or 7, wherein the composition is administered topically as compound in skincare products, preferably in sunscreen products.

9. Composition for use according to any one of claims 1 to 8, wherein the concentration of compound A is in the range of from 1 to 250 mM, preferably in the range of from 5 to 200 mM, more preferably in the range of from 10 to 100 mM, even more preferably in the range of from 25 to 50 mM.

10. Composition for use according to any one of claims 1 to 9, wherein the concentration of compound B is in the range of from 1 to 100 mM, preferably in the range of from 5 to 50 mM.

11. Composition for use according to any one of claims 1 to 10, wherein the skin cancer is skin cancer caused by ultraviolet (UV)-light irradiation, preferably a skin cancer selected from the group consisting of basal-cell carcinoma (SCC), squamous-cell carcinoma (SCC), malignant melanoma, Merkel cell carcinoma (MCC), Kaposi's sarcoma, keratoacanthoma, sebaceous carcinomas, atypical fibroxanthoma, leiomyosarcoma, angiosarcoma, and porocarcinoma.

12. Composition for use according to claim 11, wherein the skin cancer is selected from the group consisting of basal-cell carcinoma (SCC), squamous-cell carcinoma (SCC), and malignant melanoma.

13. Composition for use according to any one of claims 1 to 12, wherein the first compound A in combination with the second compound B induces melanogenesis in skin cells, especially in melanocytes, without the need of additional UV irradiation.

14. Composition for use according to any one of claims 1 to 13, wherein the composition consists of the first compound A and the second compound B.
